Europäisches Patentamt

European Patent Office

Office européen des brevets

(11) Publication number: **0 178 756**
**B1**

(12) **EUROPEAN PATENT SPECIFICATION**

(45) Date of publication of patent specification: **31.05.89**

(21) Application number: **85305582.0**

(22) Date of filing: **06.08.85**

(51) Int. Cl.⁴: **G 02 C 13/00,** A 61 L 2/02

(54) Improvements in contact lens cleaning apparatus.

(30) Priority: **29.08.84 GB 8421836**

(43) Date of publication of application:
**23.04.86 Bulletin 86/17**

(45) Publication of the grant of the patent:
**31.05.89 Bulletin 89/22**

(84) Designated Contracting States:
**CH DE FR GB IT LI NL SE**

(56) References cited:
**EP-A-0 031 152**

(73) Proprietor: **SMC Metal-Tech Company Limited
Shell Industrial Building 12 Lee Chung Street
Chai Wan (HK)**

(72) Inventor: **Yung, Simon Kwok-Choi
SHELL Ind. Build. 12 Lee Chung Street
Chai Wan (HK)**

(74) Representative: **Allen, Oliver John Richard et al
Lloyd Wise, Tregear & Co. Norman House
105-109 Strand
London, WC2R 0AE (GB)**

## Description

This invention relates to the cleaning and disinfecting of contact lenses. In particular the invention relates to apparatus in which the cleaning and disinfecting of the lenses is effected by immersing the lenses in a non-chemosterilant aqueous fluid and subjecting the lenses to ultrasonic vibrations.

To avoid eye infections and irritation it is highly desirable that contact lenses be thoroughly cleaned and disinfected at regular intervals. Some systems have proposed immersing the lenses in a cleaning or disinfecting liquid and also heating the liquid. Such systems have not proved very satisfactory in practice.

It has also been proposed to use ultrasonic vibrations to assist. An example of such apparatus is shown and described in European Patent No 31152, and comprises at least one cavity for containing at least one contact lens together with an aqueous non-chemosterilant liquid, at least one ultrasonic transducer for applying ultrasonic vibrations to the liquid and lenses contained in the cavity, oscillating means for driving the ultrasonic transducer, the oscillating means including one or more power transistors and a timer for controlling the energising of the oscillator means for a predetermined period of time.

When there is no separate heating of the fluid during the application of the ultra-sonic energy, the fluid and lens will heat up from room temperatures to about 65°C in 15 to 20 minutes and so the time of operation has to be at least 25 minutes and preferably from 30 to 40 minutes. If, however, the liquid is heated the time of operation can be reduced.

Accordingly the invention is charaterised in that heat conduction means are provided to transfer heat produced in the operation of the power transistor or transistors to the liquid in the cavity.

This has important advantages in saving energy since the time of operation can be reduced.

According to one preferred embodiment of the invention, the heat conduction means can be a strip of metal contacting the outer wall of the cavity and in contact with the transistors.

The apparatus is very effective in disinfecting lenses because the frequency of the vibrations in the cavity is resonant and largely constant which has been found to promote the killing and removal of bacteria. Typically the frequency of oscillation should be in the range of 35 to 40 KHz.

The apparatus of the invention uses a simple saline solution of the type which is widely available. No special solution or fluid is required. Thus no chemical disinfectants are necessary which has important advantages of avoiding the risk of introducing into the eye chemicals which could be dangerous or unpleasant or to which the user may be allergic. This is in contrast with some prior types of apparatus which have merely used heat to sterilise the lens which may coagulate and so deteriorate the lens coating because of the long heating time required or have merely used chemicals to sterilise the lens without the cleaning effect of the ultrasonic vibrations.

An important advantage of the invention is that the lenses are cleaned and disinfected in one step. The apparatus is therefore simple to use.

Apparatus for cleansing and disinfecting contact lenses according to the invention will now be described, by way of example, with reference to the accompanying drawings, in which:

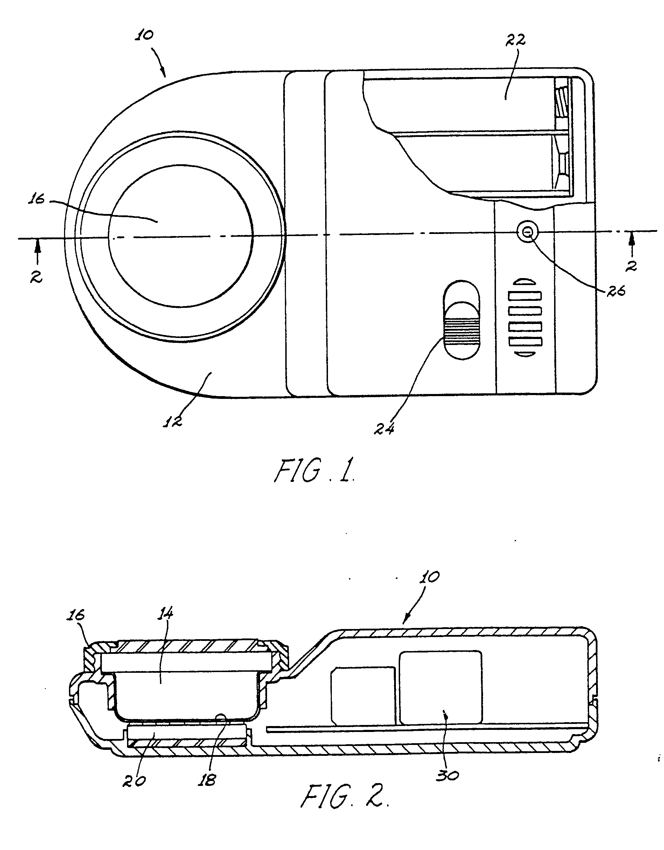

Figure 1 is a broken-away plan view of the apparatus;

Figure 2 is a section taken along the line 2-2 of Figure 1;

Figure 3 is a block diagram illustrating the operation of the apparatus;

Figure 4 is a circuit diagram of the electronic components in the apparatus;

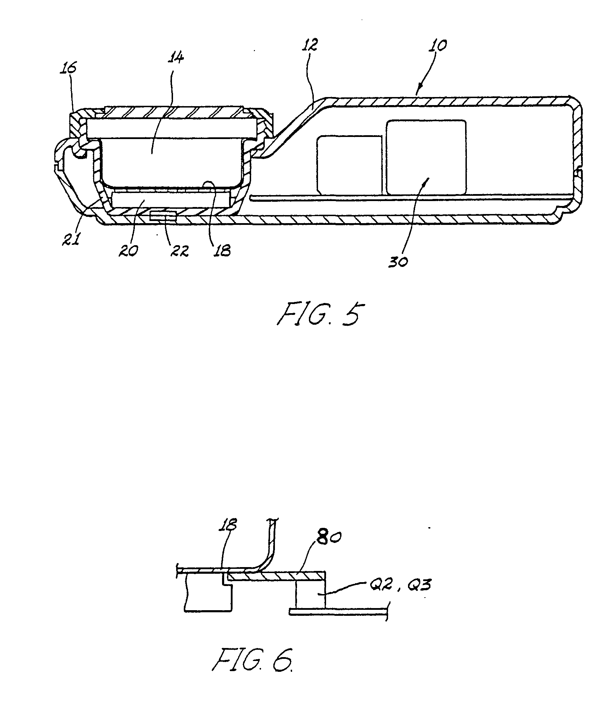

Figure 5 is a section similar to Figure 2 of a modified apparatus; and

Figure 6 is an enlarged detail section similar to Figure 2.

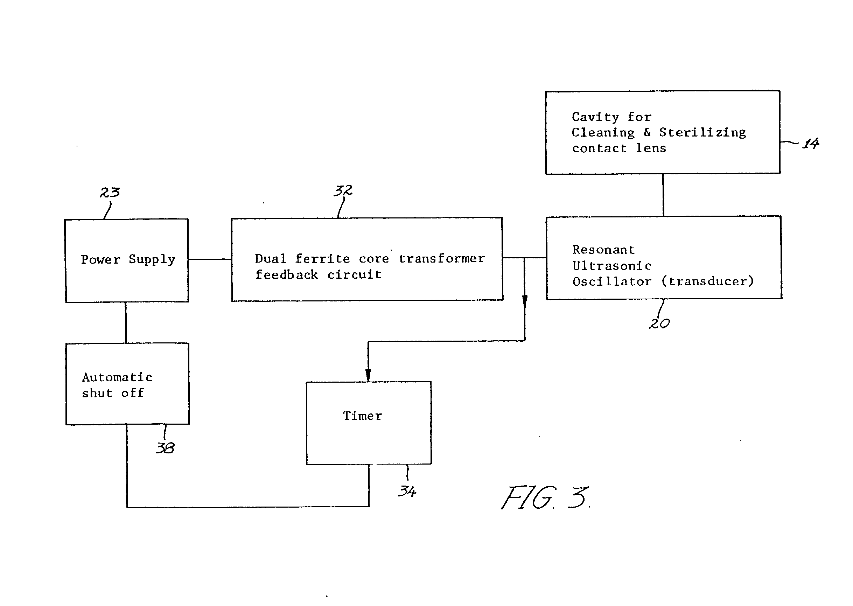

The apparatus 10 comprises a housing 12 in which is formed a cavity 14. This cavity 14 is of bowl shape as is best shown in Figure 2 and is intended to hold a pair of contact lens for cleaning and disinfecting. It is covered by a removable screw-on cap 16. The cavity has a flat base 18 and to the underside of this is adhered one or more ultra-sonic transducers 20.

The housing 12 is of compact portable size such that it can conveniently be carried in a pocket. In an alternative embodiment shown in Fugure 5, however, the cavity and screw-on cap 16 can be removed as a unit from the rest of the housing 12. Thus, the cavity and transducer 20 are enclosed in an outer bowl portion 21 which is a slidable fit within a corresponding opening in the housing 12. It will be noted that the transducer or transducers 20 are also enclosed by this portion 21 for protection. To provide electrical connection to the transducer 20, an electrical connector 22 is provided. This has 3 female sockets on the underside of the portion 20 and 3 male projecting pins upstanding from the base 12a of the housing 12. When the cavity 14, and cap 16 are in position in the housing 12, these connect and provide the required electrical connection to the transducer or transducers.

The apparatus has its own power supply 23 in the form of dry batteries contained within the housing. Also a manually operated switch 24 for controlling operation is provided together with an indicator light 26 to show when the apparatus is working.

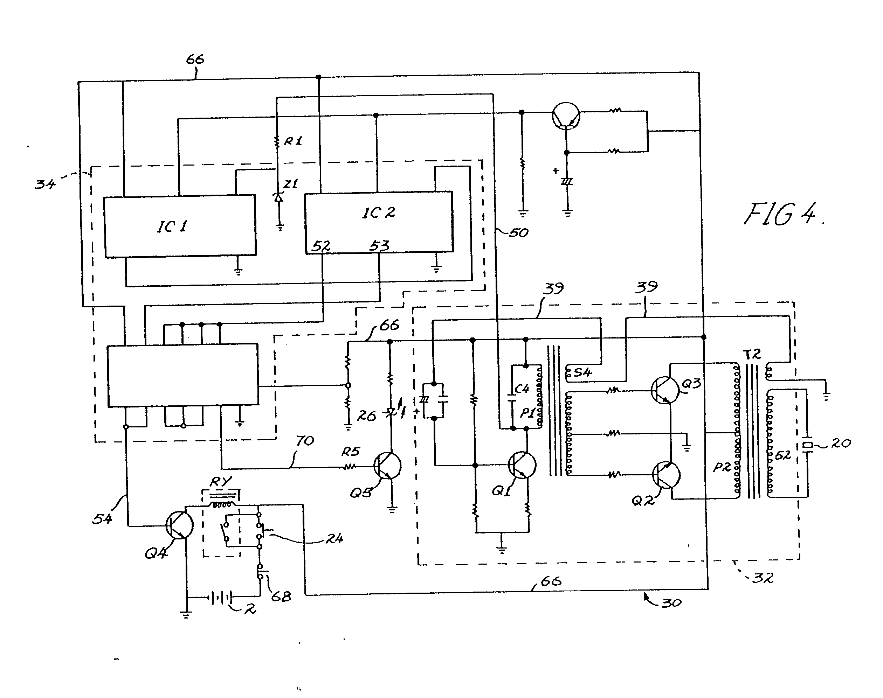

In the housing, electronic circuitry 30 for operating the transducer is provided and that circuitry 30 is shown diagrammatically in Figure 3 and in more detail in Figure 4. The circuitry 30 includes a dual ferrite core transformer feedback oscillator circuit 32 whose outPut drives the transducer 20. Such a circuit is an example of a transformer feedback circuit using a core of high magnetic permeability and low core loss. An output is taken from the circuit 32 to a timer circuit 34. Thus the timing of this circuit 34 is controlled by the oscillating frequency from the oscillator circuit. The timer in turn controls the operation of the circuit 32 by means of a shut-off switch 38 which controls the power supply 23. Preferably the circuitry 30 operates at a frequency of 35 to 40 KHz.

Thus referring to Figure 3, the user places contact lenses and saline solution in the cavity 14 and closes it with the cap 16. He then activates the circuit 32 and the ultrasonic vibrations supplied by the transducer 20 to the cavity clean and sterilise the lenses. The circuit 34 reaches a preset time and then the shut-off switch 38 disconnects the power supply 22 to the circuit 32 to terminate the operation and thereafter the user removes the cleaned lenses from the cavity 14.

As best shown in Figure 4 the oscillator circuit 32 includes two ferrite cored transformers T1 and T2. An oscillating loop is formed by a capacitor C4 and the primary coil P1 of the transformer T1 and this is driven by transistor Q1. The output of the secondary coil S1 of the transformer is used in a push-pull amplifying circuit comprising the power transistors Q2 and Q3 to drive the primary coil P2 of the transformer T2. The secondary coil S2 of the transformer T2 in turn drives the transducer 20.

Feedback is supplied by secondary coils S3 and S4 of the transformers T1 and T2, respectively, along line 39 to the transistor Q1 to ensure that the whole circuit 32 oscillates resonantly and the coils S3 and S4 couple the two transformers together. Also the resonant frequency is kept largely constant by this known arrangement irrespective of changes in the transducer 20. Thus the transducer will be affected by the amount of liquid placed in the cavity 14 and the size and type of contact lens and further by factors such as temperature and type of liquid. Despite this, the feedback in the circuit 32 ensures that the whole circuit remains in resonance and despite these changes the resonant frequency will only be insignificantly affected, e.g. by less than 500 Hz.

The circuit is also very efficient in its use of power and so an effective ultrasonic output to the cavity 14 can be provided by a power source 22 composed of dry batteries.

Because the oscillating frequency of the circuit 32 is substantially constant, it can be used to control the period of operation of the apparatus 10. Thus an output is taken along the line 50 from the oscillating loop formed by the capacitor C4 and the primary coil P1 of transformer T1 and that output is fed via resistor R1 to the counter circuit 34. This comprises integrated circuits IC1, IC2 and IC3. A zenner diode 21 limits the pulse height of the output.

The circuits IC1 and IC2 are counters which act as successive dividers of the oscillating pulses from the oscillator. There are sufficient dividing stages that after a pre-selected period of time, e.g. 9 minutes, the circuit IC2 will provide a changed output at pins 52 and 53 to the circuit IC3.

The circuit IC3 includes a logic gate and, when the circuit 30 is initially activated, the circuit IC3 provides an output along line 54, is applied to the base of a transistor Q4 which then conducts freely. As a result the relay coil 60 of a relay RY in series is activated. This has the effect of holding the switch contacts 62 of the relay RY closed so as to keep the overall circuit 30 activated from the power supply 22 via the positive line 66. When the circuit IC2 provides the changed output on pins 52 and 53, however, the output to circuit IC3 causes the latter to disable the transistor Q4 and as a result the switch contacts 64 open and the overall circuit 30 is disabled.

In parallel with the switch contacts 62 is provided the manually operated switch 24. As can be seen this has a relaxed off position. Thus when the user wishes to activate the apparatus he closes that switch temporarily to by-pass the switch contacts 62 and activate the overall circuit 30 via the line 66. The oscillating circuit 32 begins and the timer circuit 34 is reset so that in turn the transistor Q4 becomes highly conductive. The switch contacts 62 of the relay RY therefore close and so when the user releases the switch 24 actuation of the circuit continues whilst the timer circuit 34 continues to count.

A cut-out safety switch 68 is provided in series with the switch 24 and acts as a safety measure.

A further output is taken from the circuit IC3 along a line 70, through resistor R5 to a transistor Q5 in series with a light emitting diode constituting the indicator light 26. This output along line 70 enables the transistor Q5 to conduct in the same way as and simultaneously with the transistor Q4 and so all the time that the circuit is operating the transistor Q5 remains conductive and the light emitting diode 26 remains illuminated. When the circuit IC2 provides the altered output at pins 52 and 53, however, the output from the circuit IC3 along line 70 disables the transistor Q5 and so extinguishes the indicator light 26.

According to the invention, as shown in Figure 6 the waste heat from the transistors Q2 and Q3 is used to heat the liquid in the cavity 14. This is achieved by a strip 80 of heat conducting metal attached to each transistor which bears against the underside of the flat base 18 of the cavity 14. In this way the waste heat is used to heat up the liquid in the cavity more quickly and so the time of operation can be reduced. This can be important if it is desirble to heat up the liquid to relatively high temperatures, e.g. 65°C for 10 minutes. Thus simply to allow the internal heating of the fluid and lenses to reach this temperature as a result of the ultra-sonic energy may result in the lens being degraded by too long in exposure. Also the power transistors Q2 and Q3 give out a substantial amount of waste heat and this can be put to good use.

## Claims

1. A contact lens cleaning and disinfecting apparatus, comprising at least one cavity (14) for containing at least one contact lens together with an aqueous non-chemosterilant liquid, at least one ultrasonic transducer (20) for applying ultrasonic vibrations to the liquid and lenses contained in the cavity (14), oscillating means (32) for driving the ultrasonic transducer (20), the oscillator means (32) including one or more power transistors (Q2, Q3), and a timer (34) for controlling the energising of the oscillator means for a predetermined period of time, characterised in that heat conduction means (80) are provided to transfer heat produced in the operation of the power transistor or transistors (Q2, Q3) to the liquid in the cavity (14).

2. A contact lens cleaning and disinfecting apparatus as claimed in Claim 1 characterised in that the heat conduction means comprise a strip of metal (80) in contact with the transistor or transistors (Q2, Q3) and contacting the outer wall (18) of the cavity (14).

3. Apparatus as claimed in either preceding claim characterised in that the cavity (14) has a cap (16) to seal it and is itself removable together with the transducer (20) from the housing (12) of the apparatus so that lenses can be transported after cleaning and sterilising or disinfecting and before use.

**Patentansprüche**

1. Reinigungs- und Desinfektionsvorrichtung für Kontaktlinsen bestehend aus zumindest einem Hohlraum (14) zur Aufnahme von zumindest einer Kontaktlinse zusmmen mit einer wässrigen nichtchemosterilisierenden Flüssigkeit, zumindest einen Ultraschallwandler (20) zum Geben von Ultraschallschwingungen an die Flüssigkeit und an die im Hohlraum (14) enthaltenen Linsen, einer den Ultraschallwandler (20) antreibenden Schwingeinrichtung (32) mit einem oder mehreren Leistungstransistoren (Q2, Q3) und einem Taktgeber (34), durch den die Erregung der Schwingeinrichtung während einer vorbestimmten Zeitspanne gesteuert oder reguliert wird, dadurch gekennzeichnet, daß eine Wärmeleiteinrichtung (8D) vorgesehen ist, um die beim Betrieb des Leistungstransistor oder der Leistungstransistoren (Q2, Q3) erzeugte Wärme an die Flüssigkeit im Hohlraum (14) zu übertragen.

2. Reinigungs- und Desinfektionsvorrichtung nach Anspnruch 1, dadurch gekennzeichnet, daß die Wärmeleiteinrichtung einen Metallstreifen (80) aufweist, der mit dem Transistor oder den Transistoren (Q2, Q3) sowie mit der Aussenwand (18) des Hohlraums (14) in Kontakt steht bzw. kommt.

3. Vorrichtung nach einem der vorhergehenden Ansprüche 1 oder 2, dadurch gekennzeichnet, daß der Hohlraum (14) zum Verschließen desselben eine Kappe (16) besitzt und seinerseits zusammen mit dem Wandler (20) vom Gehäuse (12) der Vorrichtung entfernbar ist, so daß die Linsen nach dem Reinigen und Sterilisieren oder Desinfizieren sowie vor der Benutzung transportiert werden können.

**Revendications**

1. Appareil de nettoyage et de désinfection de lentilles de contact, comprenant au moins une cavité (14) destinée à contenir au moins une lentille de contact avec un liquide aqueux ne stérilisant pas chimiquement, au moins un transducteur ultrasonore (20) destiné à appliquer des vibrations ultrasonores au liquide et aux lentilles contenues dans la cavité (14), un dispositif oscillant (32) destiné à piloter le transducteur ultrasonore (20), le dispositif oscillant (32) comprenant au moins un transistor de puissance (Q2, Q3), et une minuterie (34) destinée à commander l'excitation du dispositif oscillant pendant une période prédéterminée, caractérisé en ce qu'un dispositif (80) de conduction de chaleur est disposé afin qu'il transfère la chaleur produite lors du fonctionnement du transistor ou des transistors de puissance (Q2, Q3) au liquide présent dans la cavité (14).

2. Appareil de nettoyage et de désinfection de lentilles de contact selon la revendication 1, caractérisé en ce que le dispositif de conduction de chaleur comporte une bande métallique (80) placée au contact du transistor ou des transistors (Q2, Q3) et placée au contact de la paroi externe (18) de la cavité (14).

3. Appareil selon l'une quelconque des revendications précédentes, caractérisé en ce que la cavité (14) a un capuchon (16) destiné à la fermer de manière étanche et peut elle-même être retirée, avec le transducteur (20), du boîtier (12) de l'appareil si bien que les lentilles peuvent être transportées après nettoyage et stérilisation ou désinfection et avant utilisation.

EP 0 178 756 B1

FIG .1.

FIG. 2.

1

FIG. 3.

Cavity for Cleaning & Sterilizing contact lens — 14

23

Power Supply

32

Dual ferrite core transformer feedback circuit

Resonant Ultrasonic Oscillator (transducer)

20

Automatic shut off

38

Timer

34

FIG 4.

EP 0 178 756 B1

FIG. 5

FIG. 6.